# FASCICULE DE BREVET EUROPEEN

(11) **EP 1 051 509 B1**
(45) Date de publication et mention de la délivrance du brevet: **02.11.2006**
(21) Numéro de dépôt: 99901643.9
(22) Date de dépôt: 28.01.1999
(51) Int. Cl.: C12Q 1/04, C12Q 1/37

(54) **DERIVES D'INDOLAMINE DANS LA DETECTION DE PEPTIDASES DE MICRO-ORGANISMES**
INDOLDERIVATE ZUM NACHWEIS VON PEPTIDASEN VON MIKROORGANISMEN
USE OF INDOLAMINE DERIVATIVES FOR DETECTING MICRO-ORGANISM PEPTIDASE

(30) Priorité: 28.01.1998 FR 9800917
(43) Date de publication de la demande: 15.11.2000
(73) Titulaire: BIO MERIEUX, 69280 Marcy l'Etoile (FR)
(72) Inventeur: ORENGA, Sylvain, F-01160 Neuville sur Ain (FR)
(74) Mandataire: Le Coupanec, Pascale A.M.P.
(86) Numéro de dépôt international: PCT/FR1999/000166
(87) Numéro de publication internationale: WO 1999/038995

(56) Documents cités:
- EP-A- 0 224 830
- DE-A- 4 119 956
- FR-A- 2 708 286
- LOJDA Z ET AL: "THE HISTOCHEMICAL DEMONSTRATION OF AMINOPEPTIDASE WITH BROMOINDOLYL LEUCINAMIDE" HISTOCHEMISTRY, vol. 43, no. 4, 1975, pages 355-366, XP000671121 cité dans la demande
- D J YARBOROUGH, O T MEYER, A M DANNENBERG, B PEARSON: "Histochemistry of Macrophage Hydrolases" JOURNAL OF THE RETICULOENDOTHELIAL SOCIETY, vol. 4, 1967, pages 390-408, XP002084546 cité dans la demande

## Description

La présente invention concerne l'utilisation d'au moins un composé à base d'un dérivé d'indolamine pour la mise en évidence d'une activité d'aminopeptidase ou de peptidase dans une culture de micro-organismes, ainsi qu'un procédé pour révéler une telle activité enzymatique, ou pour identifier des micro-organismes exprimant ou n'exprimant pas cette activité.

On appelle généralement aminopeptidase une enzyme capable de cliver par hydrolyse le groupement amide formé entre un acyle d'acide aminé et une amine primaire, et on appelle peptidase une enzyme capable de cliver par hydrolyse le groupement amide formé entre le reste acyle d'un peptide et une amine primaire. Dans la présente demande, le terme "peptidase" peut désigner, selon les cas, aussi bien une peptidase telle que définie précédemment qu'une aminopeptidase.

La détection et l'identification des micro-organismes sont très importantes notamment en médecine, dans l'industrie agro-alimentaire, et pour la surveillance de l'environnement (par exemple le contrôle de l'eau). Les micro-organismes peuvent être recherchés pour leur pathogénicité, comme indicateurs de contamination, ou dans le contrôle de procédés de fabrication.

Les techniques de détection et d'identification de micro-organismes sont actuellement basées sur la recherche de séquences nucléotidiques caractéristiques, sur la recherche d'antigènes ou d'anticorps, sur la culture en milieu sélectif ou non sélectif, ou encore sur la recherche d'activités métaboliques et notamment enzymatiques (par exemple activités d'osidases, d'estérases, de peptidases, d'oxydases, etc.).

Le plus souvent, les procédés de détection et d'identification des micro-organismes associent plusieurs de ces techniques. La culture est ainsi utilisée pour multiplier et sélectionner les micro-organismes recherchés. Afin de simplifier leur détection, il a été proposé de mettre en évidence des activités biochimiques en introduisant directement dans le milieu de culture des molécules produisant une coloration ou une fluorescence. De tels milieux sont appelés milieux de détection. Les activités biochimiques recherchées peuvent être mises en évidence par diverses méthodes telles que :
- la modification physico-chimique du milieu : par exemple changement de pH révélé à l'aide d'un indicateur coloré ou fluorescent (méthyl-umbelliférone),
- le changement du potentiel redox révélé à l'aide d'un indicateur coloré (sel de tétrazolium) ou fluorescent,
- la réaction d'une molécule produite par les micro-organismes avec un composé présent dans le milieu, conduisant à une coloration,
- ou encore l'hydrolyse de molécules libérant un composé coloré ou fluorescent (naphtol, coumarine).

Les hydrolyses détectées sont en général le résultat de l'action d'une enzyme produite par le micro-organisme sur un substrat enzymatique naturel ou synthétique. Ces activités enzymatiques sont par exemple celles des enzymes suivantes : estérases (par exemple lipases, phosphatases), osidases (β-galactosidase, β-glucuronidase, N-acétyl-hexosaminidase), peptidases (alanine-aminopeptidase, trypsinase, gélatinase), DNAses, décarboxylases, désaminases, uréases, tryptophanases, oxydases, catalases, etc.

On sait par ailleurs que les milieux gélifiés sont particulièrement bien adaptés à la culture et à l'isolement de micro-organismes à partir d'un prélèvement, ainsi qu'à la détection de micro-organismes "cibles" au sein d'un mélange de micro-organismes. Sur ces milieux, les micro-organismes forment des colonies détectables à l'oeil nu, et il est hautement souhaitable que les produits des activités biochimiques recherchées restent localisés sur leur site de production. Cela permet en effet de distinguer une colonie de ses voisines si celles-ci n'expriment pas les mêmes activités. On peut ainsi utiliser diverses méthodes détectant par exemple des changements de pH (FR-A-2 671 100), des activités d'estérases (FR-2 457 323), des activités d'osidases (FR-A-2 684 110) etc. Il est évidemment possible d'utiliser conjointement plusieurs de ces méthodes, afin de mettre en évidence plusieurs espèces ou souches, et/ou afin d'accroître la sensibilité et/ou la spécificité de la détection.

Les brevets US-B-S210022 et US-B-5358854 décrivent des substrats chromogènes sensibles à l'action de la β-galactosidase. Ces substrats dérivés du 3-hydroxy indole sont par exemple des indolyl-β-D-galactosides dont le noyau indole peut porter des substituants halogène en positions 4, 5, 6 ou 7.

De DE 41 19 956 est connue l'utilisation de la L-proline-4-nitroaniline à titre d'agent chromogénique révélateur de l'activité aminopeptidase de bactéries.

Le document EP-A-0 224 830 décrit une méthode de détection de bactéries gram-négatives dans un échantillon d'urine, dans laquelle on ajoute à l'échantillon un extrait naturel provenant de certaines espèces de crabes, puis, après incubation, on met en contact l'échantillon avec un support contenant un substrat peptidique qui comprend un groupe partant chromogène. L'extrait naturel utilisé contient une proenzyme, et si l'échantillon contient des bactéries gram-négatives en nombre suffisant, les endotoxines de ces bactéries activent la proenzyme en une enzyme capable de cliver le substrat peptidique avec formation d'un produit coloré sur le support.

On ne dispose pas actuellement de moyen, adapté aux milieux gélifiés, pour mettre en évidence des activités d'aminopeptidase et de peptidase de micro-organismes. En effet, les substrats enzymatiques utilisés jusqu'à maintenant présentent l'inconvénient de libérer des molécules colorées ou fluorescentes qui diffusent dans les milieux gélifiés, ou qui ne sont révélées que par irradiation U.V. (cas de la naphtylamine ou de l'aminocoumarine), ou qui nécessitent l'addition de réactifs (cas de la naphtylamine), ou dont la coloration est peu contrastée dans les milieux réactionnels utilisés en microbiologie (cas de la nitroaniline).

Dans la présente demande, une dénomination du type (Acide aminé)-BIA ou (Peptide)-BIA désigne un composé du type 3-Acylamino-5-bromo-indole, dont l'acyle est celui dudit acide aminé ou dudit peptide.

On sait que la L-Leucine-aminopeptidase a été mise en évidence, dans des coupes histologiques de mammifères, grâce à un substrat enzymatique, le 3-L-leucylamino-5-bromo-indole, encore appelé L-Leucine-3-(5-bromoindolamine), par abréviation L-Leu-BIA, qui produit après hydrolyse un composé coloré ; voir PEARSON et al., 1963, Lab. Invest., 12 : 712. En 1967, YARBOROUGH et al., J. Reticuloendoth. Soc., 4 : 390 ont repris la technique de PEARSON et al. dans des applications similaires (coupes histologiques). Ils ont précisé que l'ajout d'un mélange de ferricyanure et de ferrocyanure de potassium, ou de sulfate de cuivre, inhibe la réaction.

En 1975 LOJDA et HAVRANKOVA, Histochemistry, 43 : 355 proposèrent d'améliorer la méthode utilisant le substrat L--Leu-BIA par l'addition d'un mélange de sel de tétrazolium et de phénazine méthosulfate, la réaction colorée observée provenant alors de la réduction du sel de tétrazolium en formazan.

Le document WO-98/04735, publié après la date de priorité dont bénéficie la présente demande, décrit l'utilisation d'un dérivé d'acylation de la 5-bromo indolamine, l'acyle étant choisi parmi les restes leucyle et alanyle, comme agent révélateur permettant de mettre en évidence, par formation d'un produit coloré, une activité de peptidase dans une culture de micro-organismes.

Lors des travaux ayant conduit à la présente invention, on a recherché s'il était possible d'utiliser ce substrat enzymatique dans la détection de micro-organismes cultivés, notamment sur milieux gélifiés. Lors d'essais préliminaires, on a ajouté de la L-Leu-BIA ou de la L-Pro-BIA, dans un milieu couramment utilisé pour la recherche d'osidases, qui est décrit à l'exemple 1 ci-après. Il n'a pas été possible de mettre en évidence une activité de leucine-aminopeptidase ou de proline-aminopeptidase, quel que soit le micro-organisme cultivé dans ce milieu (notamment des genres *Escherichia, Klebsiella, Citrobacter, Pseudomonas, Enterococcus, Staphylococcus, Streptococcus*). L'ajout des réactifs proposés par LOJDA et HAVRANKOVA s'est traduit par une inhibition plus ou moins complète de la croissance des micro-organismes, sans permettre la révélation d'une activité de peptidase avec la L-Leu-BIA ou la L-Pro-BIA.

En revanche, si l'on ajoute, dans ce même milieu de l'exemple 1, de la 7-L-leucylamino-4-méthyl-coumarine (L-Leu-AMC) ou de la 7-L-prolylamino-4-méthyl-coumarine (L-Pro-AMC), une fluorescence (et donc une activité de leucine-aminopeptidase ou de proline-aminopeptidase) est détectée avec certains de ces mêmes micro-organismes (voir l'exemple 1). De même, dans ce milieu, avec des substrats d'osidases (5-bromo-4-chloro-3-indolyl-β-D-galactoside et 6-chloro-3-indolyl-β-D-glucuronide), on peut détecter les activités de β-galactosidase et de β-glucuronidase des micro-organismes.

Avec le milieu utilisé à l'exemple 2 ci-après, il n'a pas non plus été possible de mettre en évidence une activité de peptidase avec la L-Leu-BIA ou la L-Pro-BIA.

On a maintenant découvert que l'absence de résultats avec les dérivés d'indolamine n'était pas due à une incompatibilité avec les micro-organismes ou à une inhibition de leur multiplication en culture, mais était due essentiellement à des conditions de milieu. On a en effet découvert qu'il était possible de révéler l'activité de peptidase de micro-organismes avec les dérivés d'indolamine, en utilisant d'autres milieux de culture. Les raisons pour lesquelles certains milieux sont utilisables, et d'autres non, ne sont pas connues. Néanmoins, il est possible de déterminer et de mettre au point, par de simples essais de routine, semblables à ceux décrits dans la partie expérimentale ci-après, les milieux et/ou ingrédients qui conviennent ou qui ne conviennent pas. L'invention a donc d'abord consisté notamment à rechercher, et à montrer qu'il était possible de trouver, des milieux de culture dans lesquels les substrats de peptidases, dérivés d'indolamine, qui sont mentionnés ci-dessus, sont utilisables pour mettre en évidence les activités enzymatiques correspondantes dans une culture de micro-organismes.

On a ainsi découvert notamment qu'un milieu utilisable est le suivant, contenant :
- Extrait de levure 0,5-25 g/l
- Peptone de gélatine 0,5-25 g/l
- NaCl 0-50 g/l
et éventuellement :
- Agent régulateur de pH, quantité suffisante pour pH = 3 à 9
et/ou :
- Agent gélifiant 5-35 g/l

L'agent gélifiant est un agent gélifiant usuel, par exemple l'agar.

Le pH choisi est un pH qui convient pour le micro-organisme étudié. Dans le cas d'un milieu gélifié, le pH est de préférence de 5 à 9. On peut ajuster le pH, par exemple, avec de l'acide chlorhydrique ou du carbonate de sodium.

Si on ajoute à un tel milieu, par exemple, de la L-Pro-BIA, et si on ensemence avec des micro-organismes, on obtient des colonies brunes ou incolores suivant que les micro-organismes expriment ou non une activité de L-Proline-aminopeptidase.

Des résultats comparables ont été obtenus avec des substrats du type 3-acylamino-indole dans lesquels l'acyle est le reste acyle d'un acide aminé ou d'un peptide.

L'invention a donc pour objet l'utilisation d'au moins un composé de formule (I) :

X-NH-R (I)

dans laquelle X représente un groupe indole-3-yle éventuellement substitué et R représente le reste acyle d'un acide aminé ou d'un peptide, le groupement amine ou les groupements amines présents dans R étant éventuellement sous forme protégée, comme agent révélateur, ledit agent étant à ajouter à un milieu de culture de micro-organismes et permettant de mettre en évidence, par formation d'une coloration ou d'une fluorescence dans ledit milieu, soit une activité de peptidase dans ledit milieu de culture, soit la présence d'un micro-organisme ou d'un groupe de micro-organismes exprimant une telle activité dans ledit milieu, à l'exception de l'utilisation d'un composé de formule (I) pour lequel X représente un groupe 5-bromo-indole-3-yle et R représente un reste leucyle ou alanyle. Bien entendu, l'utilisation selon l'invention permet également, le cas échéant, de constater l'absence de l'activité de peptidase recherchée et/ou l'absence du ou des micro-organismes recherchés.

L'acide aminé ou le peptide dont R représente le reste acyle répond à la formule RCOOH.

Dans les composés de formule (I), le groupement amine, ou les groupements amines présents dans R, et en particulier le groupement amine N-terminal, peuvent être, si désiré, sous forme protégée, notamment à l'aide des groupements protecteurs temporaires des amines utilisés classiquement dans la chimie des peptides. La présence ou l'absence de tels groupements protecteurs est sans influence sur la formation d'un produit coloré ou fluorescent lorsque l'activité de peptidase recherchée est présente dans le milieu de culture étudié.

Le groupement indolyle représenté par X, dans la formule (I), peut comporter un ou plusieurs substituants en l'une au moins des positions 1, 4, 5, 6 et 7. Lesdits substituants peuvent être choisis notamment parmi les substituants halogène, alkyle inférieur, aryle, aralkyle, alkoxy inférieur et aralkoxy. Dans la présente demande, les substituants alkyle inférieur et alkoxy inférieur, de même que les groupes alkyle des substituants aralkyle et aralkoxy, comportent notamment de 1 à 4 atomes de carbone. Les substituants halogènes peuvent être le fluor, le chlore, le brome et l'iode, en particulier le chlore et/ou le brome. Les substituants aryle sont notamment des groupes phényle éventuellement substitués par exemple par des groupes alkyle inférieur, halogène ou alkoxy inférieur. Le substituant éventuellement présent en position 1 est notamment un substituant alkyle inférieur, par exemple méthyle. Parmi les groupements indole-3-yle substitués représentés par X, on peut citer notamment les dérivés 4-chloro-, 6-chloro-, 5-bromo-, 1-méthyl-, 4-méthyl-, 5-méthyl-, 4,7-diméthyl-, 4,6-dichloro-, 6,7-dichloro-, 4-chloro-5-bromo, 4,5,7-trichloro-, 4,6,7-trichloro-, 4-chloro-5-bromo-7-méthyl-, 5-méthoxy-, 5-benzyl-, 5-benzyloxy- et 5-phényl-indoles.

Dans la formule (I), lorsque le groupe R représente le reste acyle d'un acide aminé, il s'agit par exemple d'un reste alanyle, prolyle, leucyle, pyroglutamyle ou arginyle. Lorsque le groupe R représente le reste acyle d'un peptide, ce peptide peut comporter jusqu'à 10 résidus d'acides aminés, en particulier jusqu'à 5 résidus d'acides aminés, et notamment jusqu'à 3 résidus d'acides aminés. Parmi les peptides dont dérive le groupe R, on citera en particulier ceux dont le résidu d'acide aminé C-terminal est choisi parmi les résidus de leucine, d'alanine, de proline et d'arginine. On peut citer à titre d'exemple de peptide l'alanyl-phénylalanyl-proline, donnant des dérivés de formule (I) pour lesquels R représente Ala-Phé-Pro-.

Les composés de formule (I) sont solubles dans les milieux aqueux. Ils peuvent être préparés selon les méthodes habituellement utilisées en synthèse peptidique. On peut notamment faire réagir une indolamine de formule X-NH₂, X étant défini comme précédemment, avec un dérivé d'un acide aminé ou d'un peptide, ledit acide aminé ou ledit peptide répondant à la formule RH, R étant défini comme précédemment. Ledit dérivé d'acide aminé ou de peptide est un dérivé à groupement carboxyle activé et à groupement(s) amine protégé(s) à l'aide d'un groupement protecteur usuel des fonctions amine primaire. Le groupe carboxyle activé est un dérivé du groupe carboxyle qui facilite la formation d'amides par réaction avec les amines primaires, par exemple un groupe anhydride mixte. On sait que l'on peut obtenir un anhydride mixte en faisant réagir par exemple un acide avec un chloroformiate d'alkyle inférieur.

Les groupements protecteurs et leur utilisation sont bien connus ; voir par exemple R.A. BOISSONAS, Adv. in Organic Chemistry, Methods and Results, Vol.3, Interscience Publishers, 1963, pp.159 et suivantes. Dans le cas présent, on peut utiliser tous les groupements protecteurs des amines primaires utilisés notamment dans la chimie des peptides, par exemple le groupement N-Cbz (N-benzyloxycarbonyl) ou N-Boc (N-butyloxycarbonyl), dont on sait qu'ils sont des groupements protecteurs temporaires des groupements amine, ceux-ci pouvant être ensuite restaurés, si désiré, par une simple hydrolyse acide. Par réaction de l'indolamine de formule X-NH₂ avec le dérivé à groupement carboxyle activé et à groupement amine protégé, puis déprotection éventuelle du ou des groupement(s) amine, on obtient le composé de formule (I). L'indolamine de formule X-NH₂ peut elle-même être obtenue notamment selon un procédé analogue à celui décrit par MADELUNG, Liebigs Annalen der Chemie, Vol.405, pp.58-95 (1914).

L'invention concerne également un procédé de mise en évidence d'une activité de peptidase dans une culture de micro-organismes, dans lequel on ajoute au milieu de culture desdits micro-organismes un agent révélateur de formule (I) tel que défini ci-dessus, et dans lequel on recherche la formation éventuelle, dans le milieu de culture, d'un composé coloré, étant entendu que si un composé coloré s'est formé, l'activité de peptidase recherchée est présente, et elle est absente s'il n'y a pas formation d'un produit coloré. Dans le procédé de l'invention, on utilise évidemment un milieu de culture sélectionné préalablement pour son aptitude à permettre la transformation d'un substrat de formule (I) en produit coloré lorsqu'une activité de peptidase correspondante est présente. On peut utiliser notamment un milieu tel que celui mentionné ci-dessus.

Dans la présente demande, on appelle "produit coloré" un produit ayant une couleur propre lorsqu'il est éclairé, en lumière blanche par exemple, ou un produit émettant une fluorescence lorsqu'il est soumis à une irradiation par l'ultraviolet ou par la lumière visible, et le mot "coloration" désigne aussi bien la présence d'un produit coloré, ayant une couleur propre lorsqu'il est éclairé en lumière blanche, que la présence d'un produit émettant une fluorescence sous l'influence d'une radiation de longueur d'onde appropriée.

L'étape dans laquelle on recherche la formation éventuelle d'un composé coloré est évidemment mise en oeuvre, le cas échéant, au bout d'un temps de culture tel que les micro-organismes recherchés se sont suffisamment multipliés pour que l'activité de peptidase recherchée soit détectable, si elle est présente.

Les composés de formule (I) sont ajoutés, dans le milieu de culture, à une concentration suffisante pour permettre de détecter l'activité de peptidase recherchée. Pour cette détection, on recherche, soit à l'oeil nu, soit à l'aide de moyens optiques appropriés, l'apparition d'une coloration ou, éventuellement, l'apparition d'une fluorescence sous irradiation par l'ultraviolet ou par la lumière visible. Cette concentration suffisante peut être déterminée préalablement, dans chaque cas, par des expériences de routine. Le milieu de culture peut contenir par exemple de 25 à 2000 mg/L du composé de formule (I).

L'un des avantages des composés de formule (I) est que les produits colorés qu'ils forment en présence d'une activité de peptidase ne diffusent pas dans les milieux de culture gélifiés.
Ils peuvent donc être utilisés en milieu gélifié. Ils peuvent aussi, bien entendu, être utilisés en milieu liquide.

Par ailleurs, les substrats de formule (I) n'inhibent pas la multiplication des micro-organismes dans les milieux de culture. On peut donc les utiliser en les ajoutant au milieu de culture à tout moment, y compris, si désiré, avant le début de la culture, en début de culture, ou en fin de culture. Dans le cas d'un milieu gélifié, il est évidemment préférable d'introduire le composé de formule (I) lors de la préparation du milieu de culture, avant gélification dudit milieu.

L'invention concerne un procédé de détection d'un micro-organisme ou d'un groupe de micro-organismes, dans un échantillon les contenant ou susceptible de les contenir. Ce procédé comprend les étapes consistant à ajouter à un milieu de culture contenant l'échantillon au moins un composé de formule (I) tel que défini ci-dessus, à rechercher la formation éventuelle d'un produit coloré ou fluorescent dans ledit milieu, et à comparer, le cas échéant, la coloration ou la fluorescence obtenue à celle obtenue avec un échantillon authentique du micro-organisme ou du groupe de micro-organismes recherché. Dans un tel procédé, la détection est évidemment fondée sur l'expression ou l'absence d'expression, par le micro-organisme ou par le groupe de micro-organismes recherché, de l'activité de peptidase que l'agent révélateur de formule (I) utilisé permet de mettre en évidence.

Selon un mode de réalisation particulier du procédé de détection de micro-organismes selon l'invention, on peut ajouter à la culture, en outre, au moins un agent révélateur additionnel permettant de détecter, par formation d'un produit coloré ou fluorescent, une activité enzymatique, qui peut être la même que celle qui est mise en évidence à l'aide des composés de formule (I) tels que définis ci-dessus, mais qui est généralement une activité enzymatique différente de celle mise en évidence à l'aide des composés de formule (I). Il peut s'agir par exemple d'une activité d'estérase, d'osidase ou de peptidase. On peut ainsi obtenir des informations supplémentaires, en liaison avec une absence de coloration (ou de fluorescence) ou en liaison avec une coloration modifiée par rapport à la coloration obtenue avec un seul substrat enzymatique. L'autre agent révélateur choisi aura des propriétés différentes de celles des dérivés d'indolamine de formule (I) : par exemple, on choisira un autre agent révélateur capable de donner un produit réactionnel ayant une couleur différente de la couleur produite par l'indolamine de formule (I). L'autre agent révélateur (ou second agent révélateur) permettra donc de révéler, grâce à sa couleur propre, ou grâce à sa fluorescence, la présence d'une activité enzymatique dont il est spécifique. Si l'activité de peptidase révélable par les dérivés d'indolamine de formule (I) est également présente, on obtiendra une coloration modifiée, différente de la coloration donnée par le composé de formule (I) lorsqu'il est utilisé seul, et différente aussi de ladite couleur propre donnée par le second agent révélateur. Des exemples d'utilisation de plusieurs substrats, ainsi que les informations qui peuvent en ressortir, sont donnés ci-après dans la partie expérimentale. Bien entendu, les résultats peuvent varier avec chaque espèce ou souche de micro-organisme étudié. Chaque cas susceptible d'intérêt doit donc faire l'objet d'études préalables, selon des expériences de routine.

Les dérivés servant à mettre en évidence des activités enzymatiques différentes, et utilisables comme agents révélateurs additionnels, sont des agents révélateurs connus, ou leurs analogues. Ce sont notamment des dérivés d'indoxyle, de coumarine; de résorufine, de naphtol, de naphtylamine, de nitrophénol, de nitroaniline, de rhodamine, d'hydroxyquinoléine, de fluorescéine, etc.

Parmi ces agents révélateurs additionnels qui peuvent être utilisés en association avec les dérivés d'indolamine, on peut citer notamment le 5-bromo-4-chloro-3-indolyl-β-D-glucuronide, le 6-chloro-3-indolyl-β-D-glucoside, la L-alanine-7-amino-4-méthyl-coumarine, le 4-méthyl-umbelliféryl-N-acétyl-β-D-galacto-saminide, le résorufine-β-D-galactoside, le sulfate de β-naphtyle, le naphtol AS-BI β-D-galactoside, le L-alanine β-naphtylamide, l'o-nitrophénol-β-D-galactoside, le carboxyben-zoyl-L-arginine-p-nitroanilide, la rhodamine 110 bis-(L-leucine amide), l'hydroxyquinoléine-β-D-glucoside, et le diacétate de fluorescéine.

Les caractéristiques et avantages de l'invention sont illustrés par les exemples suivants.

### Exemple 1

Le milieu de culture contient, outre de l'eau :
- Peptone de viande ① 15 g/l
- Peptone de caséine ② 3 g/l
- NaCl 5 g/l
- Tampon Tris 0,5 g/l
- Na₂HPO₄ 1 g/l
- Citrate de sodium 1 g/l
- Glucose 1 g/l
- Pyruvate de sodium 2 g/l
- Agar 13 g/l
① commercialisé par : D.I.F.C.O.
② commercialisé par : D.I.F.C.O.

A ce milieu, on ajoute soit de la L-Pro-BIA, soit de la 7-L-prolyl-amino-4-méthyl-coumarine (L-Pro-AMC), à une concentration de 200 mg/l. On ensemence les différents milieux obtenus, répartis dans des boîtes de Petri, avec des micro-organismes. Les boîtes ont été mises en incubation à 37°C pendant 48 heures. Les colonies formées ont été examinées visuellement à la lumière ambiante et sous lampe U.V. (longueur d'onde = 365 nm) après 24 et 48 heures d'incubation. La couleur ou la présence de fluorescence ont été notées. Les micro-organismes étudiés étaient : *Pseudomonas aeruginosa et Candida albicans.*

Après 24 heures ou 48 heures de culture, on n'observe aucune coloration dans les cultures contenant de la L-Pro-BIA, bien que ces cultures renferment une activité de L-Pro-peptidase qui peut être mise en évidence dans les cultures contenant de la L-Pro-AMC : on observe alors l'émission d'une fluorescence sous irradiation U.V.

Quand on ajoute au milieu ci-dessus soit de la L-Ala-BIA, soit de la L-Leu-BIA, soit de la L-Alanine-7-amino-4-méthyl-coumarine (L-Ala-AMC), soit de la L-Leucine-7-amino-4-méthyl-coumarine (L-Leu-AMC), à des concentrations de 200 mg/L, on n'observe, après 24 heures ou 48 heures de culture, aucune coloration dans le cas des dérivés de bromoindolamine (BIA). En revanche, avec les dérivés d'aminométhylcoumarine (AMC), on a pu révéler la présence des activités de L-Alanine-aminopeptidase et de L-Leucine-aminopeptidase dans le cas d'*Escherichia coli, Klebsiella Pneumoniae, Citrobacter freundii, P. aeruginosa, Streptococcus agalactiae, Enterococcus faecium,* et *Streptococcus pyogenes*, et une absence de ces activités avec *Staphylococcus epidermidis.*

### Exemple 2

Dans un tampon phosphate 0,1 M pH 7,3 à 25°C on ajoute soit de la L-Pro-BIA, soit de la L-Pro-AMC, à la concentration de 400 mg/L. Le milieu obtenu est réparti dans les puits de plaques de microtitration que l'on ensemence avec des suspensions de *P. aeruginosa* ou de *C. albicans.* Les plaques sont mises en incubation pendant 24 heures à 37°C. Les puits sont examinés visuellement à la lumière ambiante et sous lampe U.V., après 24 à 48 heures d'incubation.

Aucune coloration n'a été notée avec la L-Pro-BIA, bien qu'il soit possible de mettre en évidence une activité de L-Pro-aminopeptidase dans des puits dans lesquels on a remplacé la L-Pro-BIA par la L-Pro-AMC.

De façon analogue, l'addition de L-Leu-BIA au tampon phosphate mentionné ci-dessus n'a pas permis de détecter une activité de L-Leucine-aminopeptidase dans les cultures *de E. coli, K. pneumoniae, C. freundii, P. aeruginosa, S. agalactiae, E. faecium et S. pyogenes,* alors que cette activité est détectée, par émission de fluorescence après 24 à 48 heures, pour ces mêmes bactéries, dans le même tampon additionné de L-Leu-AMC.

### Exemple 3

Le milieu de culture contient, outre de l'eau :
- Extrait de levure ① 6 g/l
- Peptone de gélatine ② 5 g/l
- NaCl 8 g/l
- Na₂CO₃ 0,1 g/l
- Agar 13 g/l
① commercialisé par BIO MERIEUX
② BIO-GELYTONE commercialisé par BIO MERIEUX

A ce milieu, on ajoute soit la L-Pro-BIA, soit la pyroglutamyl-BIA (Pyr-BIA), soit la Ala-Phe-Pro-BIA. On ensemence ces différents milieux, répartis en boîtes de Petri, avec divers micro-organismes : *E. coli, K. pneumoniae, C. freundii, P. aeruginosa, S. agalactiae, E. faecium, S. pyogenes, S. epidermidis, C. albicans et C. tropicalis.* Les boîtes sont mises en incubation à 37°C pendant 48 heures, et les colonies formées sont examinées visuellement après 24 et 48 heures d'incubation. La couleur a été notée. Les résultats sont présentés dans le tableau 1 ci-après :

**TABLEAU 1**

| **Souches** | | **L-Pro-BIA** | **Pyr-BIA** | **Ala-Phe-Pro-BIA** |
|---|---|---|---|---|
| *E. coli* | 24 H | -¹ | - | Brun² |
| *Gram -* | 48 H | - | - | Brun |
| *K. pneumoniae* | 24 H | - | - | Brun- |
| *Gram -* | 48 H | - | Brun | Brun |
| *C. freundii* | 24 H | - | - | Brun |
| *Gram -* | 48 H | - | Brun | Brun |
| *P. aeruginosa* | 24 H | Brun | Brun | Brun |
| *Gram -* | 48 H | Brun | Brun | Brun |
| *S. agalactiae* | 24 H | NT³ | - | Brun |
| *Gram +* | 48 H | NT | - | Brun |
| *E. faecium* | 24 H | NT | Brun | - |
| *Gram +* | 48 H | NT | Brun | - |
| *S. pyogenes* | 24 H | NT | Brun | Brun |
| *Gram +* | 48 H | NT | Brun | Brun |
| *S*. *epidermidis* | 24 H | NT | - | NT |
| *Gram +* | 48 H | NT | - | NT |
| *C. albicans* | 24 H | Brun | NT | NT |
| | 48 H | Brun | NT | NT |
| *C. tropicalis* | 24 H | - | NT | NT |
| | 48 H | - | NT | NT |

| | | | | |
|---|---|---|---|---|
| ¹ : - = absence de coloration ² : Brun = couleur des colonies ³ : NT = Non testé | | | | |

Dans le milieu utilisé pour cet exemple, il est donc possible de mettre en évidence des activités de L-Proline-aminopeptidase, Pyroglutamyl-aminopeptidase et Alanine-Phénylalanine-Proline-peptidase avec la L-Pro-BIA, la Pyr-BIA et la Ala-Phe-Pro-BIA, respectivement. On peut notamment distinguer les bactéries de l'espèce *E. faecium* (colonies incolores avec Ala-Phe-Pro-BIA) des bactéries du genre *Streptococcus* étudiées (coloration brune avec Ala-Phe-Pro-BIA). On peut aussi distinguer l'espèce *P. aeruginosa* (colonies brunes avec L-Pro-BIA) des autres bactéries à Gram négatif étudiées. On peut en outre distinguer les souches de levure de l'espèce *C. albicans* (colonies brunes avec L-Pro-BIA) de celles de l'espèce *C. tropicalis.*

On a obtenu des résultats identiques avec un milieu analogue liquide, sans agar.

### Exemple 4

Dans le milieu de l'exemple 3, on a incorporé soit de la L-Pro-BIA soit de la Pyr-BIA, seules ou en combinaison avec soit du 5-Bromo-4-chloro-3-indolyl-β-D-glucoside (X-Glu) soit du 6-Chloro-3-indolyl-β-D-glucoside (Z-Glu) soit du 4-Méthylumbelliféryl-β-D-glucoside (MUGl). On ensemence ces différents milieux répartis en boîtes de Petri avec des micro-organismes. Les boîtes ont été mises en incubation à 37°C pendant 48 heures, et les colonies formées ont été examinées visuellement à la lumière ambiante et sous lampe U.V. (longueur d'onde = 365nm). Les couleurs des colonies obtenues après 24 et 48 heures d'incubation sont présentées dans le tableau 2 :

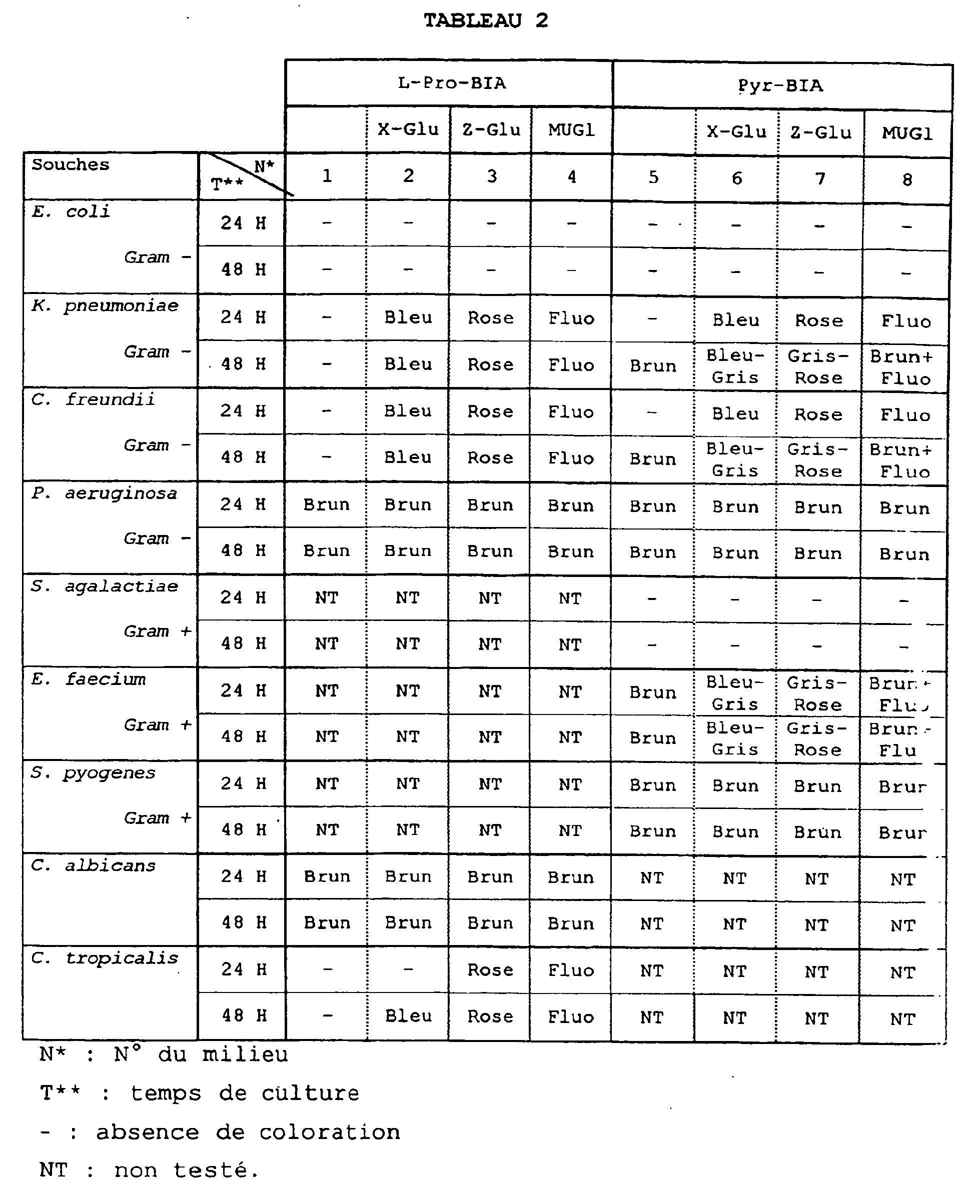

Sur les milieux 2, 3 et 4, il est possible de distinguer après 24 heures d'incubation *E. coli* (absence de coloration) et *P. aeruginosa* (coloration brune) des autres bactéries à Gram négatif. Sur ces milieux, il est également possible de distinguer *C. albicans* de *C. tropicalis,* les deux espèces produisant des colonies de couleurs différentes. Les milieux 6, 7, et 8 permettent la distinction entre *S. agalactiae* et les espèces *E. faecium* et *S. pyogenes,* les souches de *S. agalactiae* donnant des colonies incolores et celles des deux autres espèces donnant des colonies colorées. Après 24 heures d'incubation, on peut en outre, grâce à leur couleur caractéristique (variable suivant le milieu), distinguer les bactéries *E. faecium* des autres bactéries étudiées.

## Revendications

1. Utilisation d'au moins un composé de formule (I) :
X-NH-R (I)
dans laquelle X représente un groupe indole-3-yle éventuellement substitué et R représente le reste acyle d'un acide aminé ou d'un peptide, le groupement amine, ou les groupements amines, présents dans R, étant éventuellement sous forme protégée, comme agent révélateur, ledit agent révélateur étant à ajouter à un milieu de culture et permettant de mettre en évidence, par formation d'une coloration ou d'une fluorescence dans ledit milieu, soit une activité de peptidase dans une culture de micro-organismes, soit la présence d'un micro-organisme ou d'un groupe de micro-organismes exprimant une telle activité dans ledit milieu, à l'exception de l'utilisation d'un composé de formule (I) pour lequel X représente un groupe 5-bromo-indole-3-yle et R représente un reste leucyle ou alanyle.

2. Utilisation selon la revendication 1, dans laquelle R représente le reste acyle d'un acide aminé, ou le reste acyle d'un peptide comportant jusqu'à 10 résidus d'acides aminés.

3. Utilisation selon la revendication 2, dans laquelle ledit peptide comporte jusqu'à 5 résidus d'acides aminés et en particulier jusqu'à 3 résidus d'acides aminés.

4. Utilisation selon la revendication 2 ou 3, dans laquelle R représente le reste acyle du peptide Ala-Phé-Pro-.

5. Utilisation selon l'une quelconque des revendications 2 à 4, dans laquelle le résidu d'acide aminé C-terminal dudit peptide est un résidu de leucine, d'alanine, de proline ou d'arginine.

6. Utilisation selon l'une quelconque des revendications 1 et 2, dans laquelle R représente un reste alanyle, prolyle, leucyle, pyroglutamyle, ou arginyle.

7. Utilisation selon l'une quelconque des revendications précédentes, dans laquelle X représente un groupement indole-3-yle substitué en l'une au moins des positions 1, 4, 5, 6 et 7 dudit groupement indole.

8. Utilisation selon la revendication précédente, dans laquelle X représente un groupe 5-bromo-indole-3-yle.

9. Utilisation selon l'une quelconque des revendications précédentes, dans laquelle ledit milieu de culture est un milieu gélifié.

10. Utilisation selon l'une quelconque des revendications 1 à 7, dans laquelle ledit milieu de culture est un milieu liquide.

11. Utilisation selon l'une quelconque des revendications précédentes, dans laquelle on ajoute en outre au milieu de culture au moins un autre agent révélateur permettant de détecter, par formation d'un produit coloré ou fluorescent, une activité enzymatique.

12. Utilisation selon la revendication 11, dans laquelle ledit autre agent révélateur permet de détecter une activité enzymatique différente de celle mise en évidence à l'aide de l'agent révélateur de formule (I).

13. Procédé de mise en évidence d'une activité de peptidase dans un milieu de culture de micro-organismes, comprenant les étapes consistant à ajouter au milieu de culture un composé de formule (I)
X-NH-R (I)
dans laquelle X représente un groupe indole-3-yle éventuellement substitué et R représente le reste acyle d'un acide aminé ou d'un peptide, le groupement amine, ou les groupements amines, présents dans R, étant éventuellement sous forme protégée, à l'exception d'un composé de formule (I) pour lequel X représente un groupe 5-bromo-indole-3-yle et R représente un reste leucyle ou alanyle,
et à rechercher la formation éventuelle d'un produit coloré ou fluorescent dans ledit milieu, étant entendu que la formation ou l'absence de formation d'un produit coloré ou fluorescent permet de conclure à la présence ou à l'absence, respectivement, de ladite activité de peptidase.

14. Procédé de détection de la présence ou de l'absence d'un micro-organisme ou d'un groupe de micro-organismes dans un échantillon les contenant ou susceptible de les contenir, par détection de l'expression ou de l'absence d'expression par ledit micro-organisme ou groupe de micro-organismes d'une activité peptidase comprenant les étapes consistant à ajouter à un milieu de culture contenant l'échantillon un composé de formule (I)
X-NH-R (I)
dans laquelle X représente un groupe indole-3-yle éventuellement substitué et R représente le reste acyle d'un acide aminé ou d'un peptide, le groupement amine, ou les groupements amines, présents dans R, étant éventuellement sous forme protégée, à l'exception d'un composé de formule (I) pour lequel X représente un groupe 5-bromo-indole-3-yle et R représente un reste leucyle ou alanyle,
à rechercher la formation éventuelle d'un produit coloré ou fluorescent dans ledit milieu de culture,
et à comparer, le cas échéant, la coloration ou la fluorescence obtenue à celle obtenue avec un échantillon authentique du micro-organisme ou du groupe de micro-organismes recherché.

15. Procedé selon la revendication 13 ou 14, dans lequel ledit composé de formule (I) est tel que défini dans l'une quelconque des revendications 2 à 8.

16. Procedé selon la revendication 13, 14 ou 15, dans lequel on ajoute ledit composé de formule (I) au milieu de culture avant le début d'une étape de culture ou au début d'une étape de culture dudit micro-organisme ou dudit groupe de micro-organismes susceptible d'être présent dans ledit échantillon.

17. Procedé selon la revendication 16, dans lequel ledit milieu de culture est un milieu gélifié, et dans lequel on introduit le composé de formule (I) lors de la préparation du milieu de culture, avant la gélification dudit milieu.

## Claims

1. Use of at least one compound of formula (I):
X-NH-R (I)
in which X represents an optionally substituted indol-3-yl group and R represents the acyl residue of an amino acid or of a peptide, the amine group(s) present in R being optionally in protected form, as a tracer agent, said tracer agent to be added to a culture medium and making it possible to reveal, by formation of a coloration or a fluorescence in said medium, either a peptidase activity in a microorganism culture, or the presence of a microorganism or of a group of microorganisms expressing such an activity in said medium, with the exception of the use of a compound of formula (I) for which X represents a 5-bromoindol-3-yl group and R represents a leucyl or alanyl residue.

2. Use according to Claim 1, in which R represents the acyl residue of an amino acid or the acyl residue of a peptide comprising up to 10 amino acid residues.

3. Use according to Claim 2, in which said peptide comprises up to 5 amino acid residues, and in particular up to 3 amino acid residues.

4. Use according to either of Claims 2 and 3, in which R represents the acyl residue of the peptide Ala-Phe-Pro-.

5. Use according to any one of Claims 2 to 4, in which the C-terminal amino acid residue of said peptide is a leucine, alanine, proline or arginine residue.

6. Use according to either of Claims 1 and 2, in which R represents an alanyl, prolyl, leucyl, pyroglutamyl or arginyl residue.

7. Use according to any one of the preceding claims, in which X represents an indol-3-yl group substituted in at least one of positions 1, 4, 5, 6 and 7 of said indole group.

8. Use according to the preceding claim, in which X represents a 5-bromoindol-3-yl group.

9. Use according to any one of the preceding claims, in which said culture medium is a gelled medium.

10. Use according to any one of Claims 1 to 7, in which said culture medium is a liquid medium.

11. Use according to any one of the preceding claims, in which at least one other tracer agent for detecting, by formation of a coloured or fluorescent product, an enzymatic activity is also added to the culture medium.

12. Use according to Claim 11, in which said other tracer agent makes it possible to detect an enzymatic activity which is different from that revealed with the aid of the tracer agent of formula (I).

13. Method for revealing a peptidase activity in a microorganism culture medium comprising the steps consisting in adding to the culture medium at least one compound of formula (I):
X-NH-R (I)
in which X represents an optionally substituted indol-3-yl group and R represents the acyl residue of an amino acid or of a peptide, the amine group(s) present in R being optionally in protected form, with the exception of a compound of formula (I) for which X represents a 5-bromoindol-3-yl group and R represents a leucyl or alanyl residue,
and in seeking the possible formation of a coloured or fluorescent product in said medium, it being understood that the formation or absence of formation of a coloured or fluorescent product makes it possible to conclude that said peptidase activity is present or absent, respectively.

14. Method for detecting the presence or the absence of a microorganism or a group of microorganisms in a sample which contains them or which is likely to contain them, by detection of the expression or of the absence of expression by said microorganism or group of microorganisms of a peptidase activity comprising the steps consisting in adding to a culture medium containing the sample at least one compound of formula (I):
X-NH-R (I)
in which X represents an optionally substituted indol-3-yl group and R represents the acyl residue of an amino acid or of a peptide, the amine group(s) present in R being optionally in protected form, with the exception of a compound of formula (I) for which X represents a 5-bromoindol-3-yl group and R represents a leucyl or alanyl residue,
in seeking the possible formation of a coloured or fluorescent product in said culture medium,
and in comparing, where appropriate, the coloration or fluorescence obtained with that obtained with an authentic sample of the microorganism or of the group of microorganisms sought.

15. Method according to either of Claims 13 or 14, in which said compound of formula (I) is as defined in any one of Claims 2 to 8.

16. Method according to Claim 13, 14 or 15, in which said compound of formula (I) is added to the culture medium before the start of a culturing step or at the start of a culturing step for said microorganism or for said group of microorganisms which are likely to be present in said sample.

17. Method according to Claim 16, in which said culture medium is a gelled medium, and in which the compound of formula (I) is introduced during the preparation of the culture medium, before gelling of said medium.

## Patentansprüche

1. Verwendung mindestens einer Verbindung der Formel (I):
X-NH-R (I)
in der X eine gegebenenfalls substituierte Indol-3-yl-Gruppe darstellt und R den Acylrest einer Aminosäure oder eines Peptids darstellt, wobei die Aminogruppe oder die Aminogruppen, die in R vorhanden sind, gegebenenfalls in geschützter Form sind, als Entwicklungsmittel, wobei dieses Entwicklungsmittel einem Kulturmedium zuzusetzen ist und es gestattet, durch Bildung einer Färbung oder einer Fluoreszenz in diesem Medium entweder eine Peptidaseaktivität in einer Kultur von Mikroorganismen oder das Vorhandensein eines Mikroorganismus oder einer Gruppe von Mikroorganismen nachzuweisen, die eine solche Aktivität in diesem Medium exprimieren, und zwar mit Ausnahme der Verwendung einer Verbindung der Formel (I), bei der X eine 5-Bromindol-3-yl-Gruppe darstellt und R einen Leucyl- oder Alanylrest darstellt.

2. Verwendung nach Anspruch 1, bei der R den Acylrest einer Aminosäure oder den Acylrest eines Peptids mit bis zu 10 Aminosäurenresten darstellt.

3. Verwendung nach Anspruch 2, bei der das Peptid bis zu 5 Aminosäurenreste und insbesondere bis zu 3 Aminosäurenreste umfasst.

4. Verwendung nach Anspruch 2 oder 3, bei der R den Acylrest des Peptids Ala-Phe-Pro- darstellt.

5. Verwendung nach einem der Ansprüche 2 bis 4, bei der der C-terminale Aminosäurerest dieses Peptids ein Leucin-, Alanin-, Proline- oder Argininrest ist.

6. Verwendung nach einem der Ansprüche 1 und 2, bei der R einen Rest Alanyl, Prolyl, Leucyl, Pyroglutamyl oder Arginyl darstellt.

7. Verwendung nach einem der vorhergehenden Ansprüche, bei der X eine Indol-3-yl-Gruppe darstellt, die an mindestens einer der Positionen 1, 4, 5, 6 und 7 dieser Indolgruppe substituiert ist.

8. Verwendung nach dem vorhergehenden Anspruch, bei der X eine 5-Bromindol-3-yl-Gruppe darstellt.

9. Verwendung nach einem der vorhergehenden Ansprüche, bei der das Kulturmedium ein geliertes Medium ist.

10. Verwendung nach einem der Ansprüche 1 bis 7, bei der das Kulturmedium ein flüssiges Medium ist.

11. Verwendung nach einem der vorhergehenden Ansprüche, bei der man dem Kulturmedium außerdem mindestens ein anderes Entwicklungsmittel zusetzt, das es gestattet, durch Bildung eines farbigen oder fluoreszierenden Produkts eine Enzymaktivität zu erfassen.

12. Verwendung nach Anspruch 11, bei der das andere Entwicklungsmittel es gestattet, eine andere Enzymaktivität als diejenige, die mit Hilfe des Entwicklungsmittels der Formel (I) nachgewiesen wird, zu erfassen.

13. Verfahren zum Nachweis einer Peptidaseaktivität in einem Mikroorganismen-Kulturmedium, umfassend die Schritte, die darin bestehen, dass dem Kulturmedium eine Verbindung der Formel (I)
X-NH-R (I)
zugesetzt wird, in der X eine gegebenenfalls substituierte Indol-3-yl-Gruppe darstellt und R den Acylrest einer Aminosäure oder eines Peptids darstellt, wobei die Aminogruppe oder die Aminogruppen, die in R vorhanden sind, gegebenenfalls in geschützter Form sind, und zwar mit Ausnahme einer Verbindung der Formel (I), bei der X eine 5-Bromindol-3-yl-Gruppe darstellt und R einen Leucyl- oder Alanylrest darstellt,
und die eventuelle Bildung eines farbigen oder fluoreszierenden Produkts in diesem Medium gesucht wird, wobei gilt, dass die Bildung oder das Fehlen eines farbigen oder fluoreszierenden Produkts es gestattet, auf das Vorhandensein bzw. das Fehlen dieser Peptidaseaktivität zu schließen.

14. Verfahren zur Erfassung des Vorhandenseins oder Fehlens eines Mikroorganismus oder einer Gruppe von Mikroorganismen in einer Probe, die diese enthält oder enthalten kann, durch Erfassung der Expression oder des Fehlens einer Expression einer Peptidaseaktivität durch diesen Mikroorganismus oder diese Gruppe von Mikroorganismen, umfassend die Schritte, die darin bestehen, dass dem die Probe enthaltenden Kulturmedium eine Verbindung der Formel (I)
X-NH-R (I)
zugesetzt wird, in der X eine gegebenenfalls substituierte Indol-3-yl-Gruppe darstellt und R den Acylrest einer Aminosäure oder eines Peptids darstellt, wobei die Aminogruppe oder die Aminogruppen, die in R vorhanden sind, gegebenenfalls in geschützter Form sind, und zwar mit Ausnahme einer Verbindung der Formel (I), bei der X eine 5-Bromindol-3-yl-Gruppe darstellt und R einen Leucyl- oder Alanylrest darstellt,
die eventuelle Bildung eines farbigen oder fluoreszierenden Produkts in diesem Kulturmedium gesucht wird
und gegebenenfalls die erhaltene Färbung oder Fluoreszenz mit derjenigen verglichen wird, die mit einer authentischen Probe des gesuchten Mikroorganismus oder der gesuchten Gruppe von Mikroorganismen erhalten wird.

15. Verfahren nach Anspruch 13 oder 14, bei dem die Verbindung der Formel (I) so ist, wie sie in einem der Ansprüche 2 bis 8 definiert ist.

16. Verfahren nach Anspruch 13, 14 oder 15, bei dem man die Verbindung der Formel (I) dem Kulturmedium vor dem Beginn eines Schritts der Kultur oder zu Beginn eines Schritts der Kultur dieses Mikroorganismus oder dieser Gruppe von Mikroorganismen zusetzt, der bzw. die in dieser Probe vorliegen kann.

17. Verfahren nach Anspruch 16, bei dem das Kulturmedium ein geliertes Medium ist und bei dem man die Verbindung der Formel (I) bei der Zubereitung des Kulturmediums vor der Gelierung dieses Mediums einführt.
